(19) Europäisches Patentamt

European Patent Office

Office européen des brevets

(11) **EP 1 014 925 B1**

(12) **EUROPÄISCHE PATENTSCHRIFT**

(45) Veröffentlichungstag und Bekanntmachung des Hinweises auf die Patenterteilung:
**21.09.2005 Patentblatt 2005/38**

(21) Anmeldenummer: **98903980.5**

(22) Anmeldetag: **27.02.1998**

(51) Int Cl.⁷: **A61K 7/42**

(86) Internationale Anmeldenummer:
**PCT/CH1998/000083**

(87) Internationale Veröffentlichungsnummer:
**WO 1998/038977 (11.09.1998 Gazette 1998/36)**

(54) **KOMBINATION VON ERYTHRULOSE UND EINEM REDUZIERENDEN ZUCKER MIT SELBSTBRÄUNUNGSEIGENSCHAFTEN**

COMBINATION OF ERYTHRULOSE AND A REDUCING SUGAR WITH SELF-BROWNING PROPERTIES

COMBINAISON D'ERYTHRULOSE ET D'UN SUCRE REDUCTEUR PRESENTANT UN EFFET AUTOBRONZANT

(84) Benannte Vertragsstaaten:
**AT CH DE ES FR GB IT LI LU MC**

(30) Priorität: **05.03.1997 CH 51897**

(43) Veröffentlichungstag der Anmeldung:
**05.07.2000 Patentblatt 2000/27**

(73) Patentinhaber: **PENTAPHARM A.G.**
**CH-4002 Basel (CH)**

(72) Erfinder:
- **SCHREIER, Thomas**
**CH-4416 Bubendorf (CH)**
- **JERMANN, Roland**
**CH-4242 Laufen (CH)**

(74) Vertreter: **Braun, André jr.**
**Braunpat Braun Eder AG**
**Patent - Marken - Rechtsanwälte**
**Reussstrasse 22**
**Postfach**
**4015 Basel (CH)**

(56) Entgegenhaltungen:
**EP-A- 0 576 189          WO-A-95/22960**
**FR-A- 1 307 259**

- **"Simulated sun - taking the heat out ot tanning" MANUFACTURING CHEMIST, Bd. 63, Nr. 7, 1992, WOOLWICH, LONDON (GB), Seiten 18-21, XP000290706**

**Beschreibung**

Die vorliegende Erfindung betrifft eine Kombination von Erythrulose in D- oder L-Form oder als Racemat und mindestens einem weiteren reduzierenden Zucker mit Selbstbräunungseigenschaften und diese enthaltende kosmetische Zusammensetzungen zur künstlichen Bräunung der Haut. Die kosmetischen Zusammensetzungen bewirken eine gleichmässigere und länger andauernde Färbung der Haut und vermindern deren Austrocknung und unregelmässige Abschuppung verglichen mit bekannten Zusammensetzungen, welche z.B. Dihydroxyaceton allein enthalten.

Hydroxyketone und Hydroxyaldehyde sind bekannte Wirkstoffe für Selbstbräuner. Die in der Regel in Cremes verarbeiteten Wirkstoffe reagieren mit freien Aminogruppen des Stratum corneum und ergeben bräunlich gefärbte Farbstoffe, welche an Proteine des Stratum corneum gebunden sind. Diese als Maillard-Reaktion bekannte Umsetzung von reduzierenden Zuckern mit Aminosäuren, Peptiden oder Proteinen führt zu Verbindungen mit Carbonylfunktion, welche zu braunen Melanoiden polymerisieren.

Der bis heute am häufigsten eingesetzte Wirkstoff ist Dihydroxyaceton (im nachfolgenden "DHA" genannt ; vgl. z.B. EP-A-0576189). Der schnell bräunende Wirkstoff (4-7 Stunden) weist aber einige Nachteile auf. Die erzielte Hautfarbe ist gelb-braun, was vor allem bei heller Haut zu einer unnatürlichen Farbe führt. Ferner wird die Bräunung unregelmässig und verblasst bald wieder, weil die obersten Schichten der Epidermis schnell abgetragen werden.

Es wurde nun gefunden, dass eine Kombination von DHA - oder einem andern reduzierenden Zucker mit ähnlichen Eigenschaften - mit Erythrulose die obigen Nachteile nicht aufweist. Dies lässt sich darauf zurückführen, dass Erythrulose die Haut langsamer (die Farbentwicklung dauert etwa 20-24 Stunden) und weniger intensiv färbt, dafür aber auch in tiefere Schichten des Stratum corneum eindringen und auch dort mit den freien Aminogruppen reagieren kann. Dadurch resultiert nebst gleichmässigerer Bräunung auch ein Retardeffekt, so dass die Bräunung länger anhält. Zudem wird die Haut weniger ausgetrocknet als z.B. mit DHA alleine. Die anhand von Beispielen später noch dargestellte synergistische Wirkung der Wirkstoffkombination Erythrulose/ weiterer reduzierender Zucker, wie z. B. DHA, war nicht vorhersehbar.

Erythrulose kann an sich auch als alleiniger Wirkstoff für leicht bräunende Tagescrèmen verwendet werden. Der Vorteil von Erythrulose gegenüber anderen reduzierenden Zuckern mit Selbstbräunungseffekt, insbesondere DHA, liegt darin, dass die Haut gleichmässiger und ohne Bildung von Bräunungsrändern gebräunt wird.

Erythrulose ist ein C4-Ketozucker der allgemeinen Strukturformel 1,3,4-Trihydroxy-butan-2-on, der natürlich vorkommt oder auf an sich bekannte Weise chemisch oder biotechnologisch hergestellt werden kann. Erythrulose kann in D- oder L-Form oder auch als Racemat benutzt werden.

DHA ist ein C3-Ketozucker der allgemeinen Strukturformel 1,3-Dihydroxy-propan-2-on, der auf an sich bekannte Weise hergestellt werden kann.

Weitere in Kombination mit Erythrulose verwendbare reduzierende Zucker mit Selbstbräunungseigenschaften sind z.B. Glucose, Xylose, Fructose, Reose, Ribose, Arabinose, Allose, Tallose, Altrose, Mannose, Galactose, Laktose, Sucrose, Erythrose und Glyceraldehyd.

Die Wirkstoffkombination enthält ein Verhältnis zwischen Erythrulose und reduzierendem Zucker von etwa 10 : 1 bis 1 : 10, vorzugsweise etwa ca. 1 : 1 bis 1 : 3, besonders bevorzugt 1 : 2.

In einer kosmetischen Formulierung verwendet man etwa 1-5% Erythrulose und etwa 1-15% reduzierenden Zucker, vorzugsweise etwa 1,5% Erythrulose und 3,5% reduzierenden Zucker.

Die Stabilität von Erythrulose mit einem reduzierenden Zucker in einer kosmetischen Formulierung hängt von vielen Faktoren ab. Die Wirkstoffkombination kann sowohl in O/W-(z.B. Creme oder Lotio) als auch W/O-Emulsionen und weiteren kosmetischen Formulierungstypen (z.B. multiple Emulsionen, wie O/W/O- oder W/O/W-Emulsionen, Gele, Salben, Aerosole) eingesetzt werden. Die Stabilität der Wirkstoffe kann chromatographisch quantitativ bestimmt werden. Dazu wird die Emulsion verdünnt, membranfiltriert und mittels HPLC analysiert.

Die ölige Phase in Emulsionen kann z.B. KW-Öle wie Paraffinöl oder Mineralöle, Wachse wie Bienenwachs oder Paraffinwachs, Siliconöle wie Cyclomethicone oder Dimethicone, Fettalkohole wie Stearylalkohol oder Cetylalkohol, natürliche Öle wie Jojobaöl, Sesamöl oder Sonnenblumenöl, Fettsäureester wie Isopropylmyristat oder Glycerylstearat oder Mono-, Di- oder Triglyceride, wie z.B. Capryl/Caprinsäure-Triglycerid, enthalten.

Bei Öl-in-Wasser-Emulsionen umfasst die Ölphase vorzugsweise 5-45 Gew.-%, insbesondere etwa 10-30 Gew.-% der Gesamtformulierung.

Als Emulgatoren können für Öl-in-Wasser- und Wasser-in-Öl-Emulsionen an sich bekannte Emulgatoren verwendet werden, wie z.B. Silicone, Sesquioleate, Sorbitanester, alkoxylierte Sorbitan- und Fettsäureester, alkoxylierte Mono-, Di- und Triglyceride, gegebenenfalls alkoxylierte Polymere wie Crosspolymere aus Ethylenoxid und Propylenoxid, gegebenenfalls alkoxylierte Fettalkohole, Fettsäuren, Ester von natürlichen Ölderivaten, Ether wie Polyethylenglykol(n)stearylether und Polyethylencetyl(n)-stearylether.

Die Emulgatoren werden vorzugsweise in Mengen von 0,1-15 Gew.% bezogen auf die Gesamtformulierung verwendet.

Die erfindungsgemässen Formulierungen können noch weitere Hilfsstoffe enthalten, wie z.B. Stabilisatoren wie Mannit oder Cyclodextrin, Feuchthaltemittel wie Glycerin und Propylenglykol, Verdickungsmittel wie Acrylsäurepolymere oder Cellulosederivate, Antioxidantien wie Tocopherylacetat, pH-Korrigentien wie Natriumphosphat, Filmbildner wie PVP, Konservierungsmittel wie Phenoxyethanol und Paraben, Farbstoffe, Duftstoffe, Weichmacher, Antiseptika, Bakterizide, Vitamine, Pigmente, Treibmittel, sowie andere Verbindungen, die kosmetisch oder medizinisch wünschenswert sind. Beispiele solcher weiteren Verbindungen können im CTFA International Cosmetic Ingredient Dictionary, 6. Ausgabe, The Cosmetic, Toiletry and Fragrance Association, Inc., Washington D.C., 1995 gefunden werden.

Lipophile Systeme eignen sich ganz allgemein besser für kosmetische Formulierungen von Selbstbräunern. Mit der Zugabe von Penetrationsbeschleunigern kann die Bräunung beschleunigt und die Farbintensität signifikant erhöht werden. Als geeignete Penetrationsbeschleuniger haben sich vor allem Dimethicon, Cyclomethicon, Propylenglykoldipelargonat, Propylenglykol oder Ethoxydiglycol erwiesen. Die gemessene Farbintensität war bei Penetrationsbeschleunigern enthaltenden Formulierungen bis zu 55% höher im Vergleich zur Grundformulierung ohne Penetrationsbeschleuniger.

Synthetische Öle wie Miglyol 812, Isoppropylpalmitat und siliconische Öle und natürliche Lipide wie Jojobaöl und Sesamöl vertragen sich besonders gut mit der Wirkstoffkombination und werden deshalb bevorzugt verwendet.

Auch Gele können als Formulierungsgrundlage für die erfindungsgemässen Zusammensetzungen verwendet werden. Als gelbildende Substanzen können Carbomere, Cellulosederivate und andere Gelbildner enthalten sein.

Sofern die kosmetische Formulierung sowohl einen Selbstbräunungseffekt als auch Schutz gegen Sonnenstrahlen bieten soll, kann man der Formulierung auch einen oder mehrere Sonnenschutzfilter, wie z.B. Octylmethoxycinnamat und/oder Butylmethoxydibenzoylmethan zusetzen.

Bei der Verarbeitung der Wirkstoffe zu kosmetischen Formulierungen ist zu beachten, dass die Wirkstoffe nur bei niedrigeren Temperaturen von etwa <40°C zugefügt werden und dass der pH-Wert 5 in den kosmetischen Formulierungen nicht überschritten wird, was durch Pufferung leicht bewerkstelligt werden kann.

Die erfindungsgemässe Wirkstoffkombination resp. die darauf basierenden kosmetischen Formulierungen können auf übliche Art auf die menschliche Haut aufgetragen werden.

Die Erfindung wird im folgenden an Hand von Beispielen näher erläutert, wobei die Prozentangaben jeweils Gew.-% darstellen.

Beispiel 1

Es wird eine selbstbräunende Körperlotio hergestellt, indem man eine ölige Phase aus 12,00 % Cutina GMS (Glycerylstearat), 1,50 % Eumulgin B1 (Ceteareth-12), 1,50 % Eumulgin B2 (Ceteareth-20), 4,00 % Isopropylmyristat, 7,00 % Paraffinöl und 4,00 Miglyol 812 (Capryl/Caprinsäure-Triglycerid) miteinander mischt und auf 70°C erwärmt und eine wässrige Phase aus 51,20 % demineralisiertem Wasser, 0,50 % Phenonip und 3,00 % Glycerin miteinander mischt und auf 75°C erwärmt. Die wässrige Phase wird darauf unter Rühren der öligen Phase zugegeben und auf 50°C abgekühlt, homogenisiert und weiter auf 30°C abgekühlt und dem Gemisch eine weitere wässrige Phase aus 10,00 % demineralisiertem Wasser, 1,50 % Erythrulose und 3,50 % Dihydroxyaceton zugegeben und kalt gerührt. Es entsteht dabei eine angenehm verwendbare Körperlotio.

Beispiel 2

Es wird eine selbstbräunende weisse Lotio hergestellt, indem man eine ölige Phase aus 1,50 % Arlatone 983 S (PEG-5 Glycerylstearat), 2,20 % Arlatone 985 (PEG-5 Glycerylstearat), 1,50 % Brij 76 (Steareth-10), 5,00 % Miglyol 812 (Capryl/Caprinsäure-Triglycerid), 4,00 % Paraffinöl und 1,00 % Siliconöl AK 500 (Dimethicone) miteinander mischt und auf 70°C erwärmt und eine wässrige Phase aus 59,30 % demineralisiertem Wasser, 0,50 % Phenonip und 10,00 % Propylenglykol miteinander mischt und auf 75°C erwärmt. Die wässrige Phase wird darauf unter Rühren der öligen Phase zugegeben und auf 50°C abgekühlt, homogenisiert und weiter auf 30°C abgekühlt und dem Gemisch eine weitere wässrige Phase aus 10,00 % demineralisiertem Wasser, 1,50 % Erythrulose und 3,50 % Dihydroxyaceton zugegeben und kalt gerührt. Es entsteht dabei eine angenehm verwendbare Lotio.

Beispiel 3

Es wird analog Beispiel 2 eine selbstbräunende weisse Lotio hergestellt mit der Ausnahme, dass man 3,00 % Erythrulose und 2,00 % DHA verwendet. Es entsteht dabei wie in Beispiel 2 eine angenehm verwendbare Lotio.

Beispiel 4

Es wird eine selbstbräunende Creme hergestellt, indem man eine ölige Phase aus 1,00 % Cremophor A6 (Ceteareth 6 und Stearylalkohol), 1,00 % Cremophor A25 (Ceteareth 25), 3,00 % Cutina GMS (Glycerylstearat), 10,00 % Paraffinöl, 5,00 % Jojobaöl und 1,00 % Cetylalkohol miteinander mischt und auf 70°C erwärmt und eine wässrige Phase aus 48,50 % demineralisiertem Wasser, 0,50 % Phenonip und 5,00 % Propylenglykol miteinander mischt und auf 75°C erwärmt. Die wässrige Phase wird darauf unter Rühren der öligen Phase zugegeben und auf 50°C abgekühlt, homogenisiert und weiter auf 30°C abgekühlt und dem Gemisch eine weitere wässrige Phase aus 15,00 % demineralisiertem Wasser, 5,00 % Cyclodextrin, 1,50 % Erythrulose und 3,50 % Dihydroxyaceton zugegeben und kalt gerührt. Es entsteht dabei eine angenehm verwendbare Creme.

Beispiel 5

Es wird eine selbstbräunende O/W-Crème mit Sonnenschutzfilter hergestellt, indem man eine ölige Phase aus 5,00 % eines Gemisches von Glycerylstearat und PEG-100-stearat, 1,00 % Cetylalkohol, 2,00 % Stearinsäure, 4,00 % Dimethicon, 2,00 % Cyclomethicon, 3,00 % Capryl-/Caprinsäure-Triglycerid, 1,00 % Jojobaöl, 4,00 % Isopropylpalmitat, 2,00 % Octylmethoxycinnamat und 1,00 % Butylmethoxydibenzoylmethan miteinander mischt, auf 75°C erwärmt und eine auf 80°C erwärmte wässrige Phase bestehend aus 51,20 % demineralisiertem Wasser, 0,50 % Phenonip, 6,00 % Propylenglykol und 2,00 % Glycerin beigibt und gut rührt. Die erhaltene Emulsion wird auf 50°C abgekühlt und homogenisiert. Bei 30°C wird dann eine weitere wässrige Phase aus 10,00 % demineralisiertem Wasser, 1,50 % Erythrulose und 3,50 % Dihydroxyaceton zugegeben und gut gerührt.

Beispiel 6

Es wird eine selbstbräunende W/O/W-Crème hergestellt, indem man eine wässrige Lösung aus 2,00 % Poloxamer, 0,10 % Phenonip, 16,02 % demineralisiertem Wasser, 0,38 % Magnesiumsulfat-Heptahydrat, 1,40 % Xanthangummi, 0,10 % Phenonip, und 20,00 % demineralisiertem Wasser vorlegt und 60,00 % einer zuvor hergestellten W/O-Emulsion bestehend aus einer Ölphase mit 3,30 % eines Gemisches aus Sorbitanester und Polyglycerinester, 3,00 % Polyglycerinester, 15,00 % Isohexadecan, 14,00 % Capryl-/Caprinsäure-Triglycerid und einer wässrigen Phase mit 55,50 % demineralisiertem Wasser, 0,20 % Phenonip, 0,70 % Magnesiumsulfat-Heptahydrat, 2,50 % Erythrulose und 5,80 % DHA beigibt und homogenisiert.

Beispiel 7

Es wird ein selbstbräunendes Gel hergestellt, indem man 1,00 % PVM (Copolymer aus Vinylchlorid und Vinylmethylether) /MA-Decadien-Crosspolymer, 0,50 % Phenonip und 83,50 % demineralisiertes Wasser miteinander mischt, mit Natronlauge den pH auf 3,7 einstellt und 15,00 % einer Mischung aus 66,70 % demineralisiertem Wasser, 10,00 % Erythrulose und 23,30 % DHA zugibt.

Beispiel 8

Es wird eine selbstbräunende Tagescrème hergestellt, indem man 5,00 % Arlacel 165 (Glycerylstearat, PEG-100-stearat, 1,00 % Cetylalkohol, 2,00 % Stearinsäure, 4,00 % Dow Corning 200 Silicone (Dimethicone-Polymethylsiloxan), 2,00 % Belsil CM 020 (Cyclomethicone), 3,00 % Miglyol 812 (Capryl/Caprinsäure-Triglycerid), 1,00 % Jojobaöl, 4,00 % Isopropylpalmitat, 2,00 % Parsol MCX (Octylmethoxycinnamat) und 1,00 % Butylmethoxydibenzoylmethan miteinander mischt und diese ölige Phase auf 70°C erhitzt. Parallel dazu mischt man 57,20 % deionisiertes Wasser, 0,50 % Phenonip, 6,00 % Propylenglykol und 2,00 % Glycerin und erhitzt diese wässrige Phase auf 75°C. Die wässrige Phase wird dann unter Rühren der öligen Phase zugegeben und die Mischung auf 50°C abgekühlt, homogenisiert und weiter auf 30°C abgekühlt. Darauf wird ein Gemisch aus 5,00 % deionisiertem Wasser und 4.00 % Erythrulose zugegeben und kalt gerührt, worauf man schliesslich das ganze Gemisch mit 0,30 % Belamie 0/241710 versetzt.
Auf diese Art erhält man eine Tagescrème, die bei täglicher Applikation eine leicht bräunliche Färbung und gesundes Aussehen der Haut zur Folge hat. Die Sonnenschutzfilter schützen die Haut zugleich vor den Auswirkungen schädlicher UV-Strahlen.

Beispiel 9

Um die synergistische Wirkung von Erythrulose und DHA auf die Bräunung der Haut zu untersuchen, wurde eine vergleichende Bräunungsstudie mit einer 1,5 % Erythrulose und 3,5 % DHA enthaltenden O/W-Lotio gemäss Beispiel 1 mit einer DHA enthaltenden Lotio durchgeführt.
Auf dem Rücken von je 4 weiblichen und männlichen Probanden wurden 10 x 14 cm grosse Felder markiert. Die Probanden durften während der ganzen Versuchszeit weder Sonnenbaden noch ein Solarium besuchen.
Die Probanden wurden vor den Messungen 15 min. in einem Hautlabor bei 26-28°C und 40-50 % Luftfeuchtigkeit einklimatisiert. Unmittelbar nach der Bestimmung des Ausgangswertes am Tag 0 wurden die markierten Versuchsfelder mit 0,3 ml der entsprechenden Lotio unter leichtem Einreiben behandelt. Dieser Ablauf wurde während den folgenden 9 Tagen wiederholt. Nach dem 10. Tag wurde keine Lotio mehr appliziert.

Vom Starttag bis zum 20. Tag wurde sowohl die Farbveränderung als auch die Hautfeuchtigkeit jeweils unmittelbar vor der nächsten Applikation gemessen. Die Hautfeuchtigkeit wurde mit dem Corneometer CM 820 (Courage und Khazaka) bestimmt. Die Hautfarbe wurde mit dem Minolta Chromameter CR 300 gemessene.

Die Hautfarbe wurde mit dem "CIE L*a*b* Farbsystem" quantifiziert. Dabei werden die Farben in einem dreidimensionalen Farbkoordinatensystem definiert. Der a*-Wert entspricht der rot-grün Achse, der b*-Wert der gelb-blau Achse und der L*-Wert der hell-dunkel Achse. Je kleiner der L*-Wert ist, desto dunkler ist die Farbe. Die Differenz zweier Messpunkte in diesem dreidimensionalen Farbenkoordinatensystem kann aus den Absolutwerten durch die folgende Formel berechnet werden:

$$\Delta E(CIE\ L^*a^*b^*) = \sqrt{(L_1^* - L_2^*)^2 + (a_1^* - a_2^*)^2 + (b_1^* - b_2^*)^2}$$

Der mit dieser Formel berechnete Wert ist eine Masszahl für die Farbveränderung der Haut.

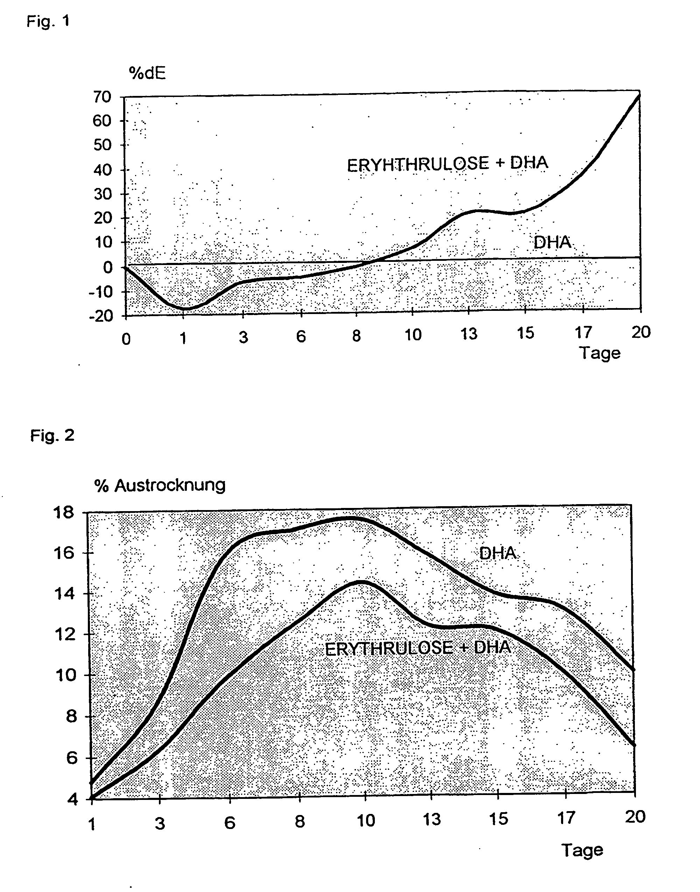

Der unterschiedliche Einfluss der beiden getesteten Formulierungen auf die Farbintensität wird in Figur 1 gezeigt. Im Diagramm ist der prozentuale Farbunterschied der Kombination von Erythrulose und DHA im Vergleich zu DHA dargestellt. Die Anwendung von DHA allein färbt die Haut in den ersten Tagen schneller. Vom 10. Tag an hingegen war die Färbungsintensität der Kombination Erythrulose und DHA grösser. Nach Beendigung der Anwendung wurde der Unterschied der Färbungsintensität der Kombination von Erythrulose und DHA im Vergleich zu DHA allein auf Grund der verzögerten Abnahme der Färbung immer ausgeprägter. Dies zeigt, dass Erythrulose einen sichtbaren Retardeffekt erzeugt.

Die mit Erythrulose und DHA erzeugte Hautfärbung zeigt keine Streifenbildung und die Haut keine Abschuppung auf Grund verstärkter Austrocknung.

Durch die Anwendung von Erythrulose und DHA in einer O/W-Emulsion wird die Haut messbar bis 30% weniger ausgetrocknet (Fig. 2). Die Haut fühlt sich weniger trocken an. Die Testpersonen empfanden bei der Anwendung der Kombination von Erythrulose und DHA im Vergleich zur DHA-Emulsion kein gespanntes Hautgefühl.

## Patentansprüche

1. Wirkstoffkombination zur künstlichen Bräunung der Haut enthaltend eine Kombination von Erythrulose in D- oder L-Form oder auch als Racemat mit einem weiteren reduzierenden Zucker mit Selbstbräunungseigenschaften.

2. Wirkstoffkombination nach Patentanspruch 1, **dadurch gekennzeichnet, dass** das Verhältnis zwischen Erythrulose und dem weiteren reduzierenden Zucker mit Selbstbräunungseigenschaften zwischen 10 : 1 bis 1 : 10, vorzugsweise etwa bei 1 : 1 bis 1 : 3, besonders bevorzugt bei ca. 1 : 2 liegt.

3. Wirkstoffkombination nach Patentanspruch 1 oder 2, **dadurch gekennzeichnet, dass** als weiterer reduzierender Zucker mit Selbstbräunungseigenschaften DHA verwendet wird.

4. Kosmetische Formulierung enthaltend eine Wirkstoffkombination nach einem der Patentansprüche 1 oder 2, **dadurch gekennzeichnet, dass** 1-5% Erythrulose und 1-15% eines weiteren reduzierenden Zuckers mit Selbstbräunungseigenschaften, vorzugsweise etwa 1,5% Erythrulose und 3,5% eines weiteren reduzierenden Zuckers mit Selbstbräunungseigenschaften verwendet werden.

5. Kosmetische Formulierung enthaltend eine Wirkstoffkombination nach einem der Patentansprüche 1 oder 2, **dadurch gekennzeichnet, dass** als weiterer reduzierender Zukker mit Selbstbräunungseigenschaften DHA verwendet wird.

6. Kosmetische Formulierung nach einem der Patentansprüche 4 oder 5 in Form einer O/W-Emulsion.

7. Kosmetische Formulierung nach Patentanspruch 6, **dadurch gekennzeichnet, dass** die ölige Phase 5-45 Gew.-%, insbesondere etwa 10-30 Gew.-% der Gesamtformulierung beträgt.

8. Kosmetische Formulierung nach einem der Patentansprüche 4-7, **dadurch gekennzeichnet, dass** sie mindestens einen Penetrationsbeschleuniger enthält.

9. Kosmetische Formulierung nach einem der Patentansprüche 4-8, **dadurch gekennzeichnet, dass** sie mindestens eine als Sonnenschutzfilter wirkende Verbindung, vorzugsweise Octylmethoxycinnamat und/oder Butylmethoxydibenzoylmethan, enthält.

10. Nichttherapeutisches Verfahren zur topischen Verwendung einer Wirkstoffkombination gemäss einem der Patentansprüche 1-3 oder einer kosmetischen Formulierung gemäss einem der Patentansprüche 4-9, indem eine wirksame Menge einer Wirkstoffkombination oder einer kosmetischen Formulierung topisch auf die Haut aufgetragen wird.

11. Verwendung einer Wirkstoffkombination gemäss einem der Patentansprüche 1-3 zur Herstellung einer kosmetischen Formulierung gemäss einem der Patentansprüche 4-9.

12. Verwendung einer kosmetischen Formulierung gemäss einem der Patentansprüche 4-9 für die künstliche Bräunung der Haut, indem die Formulierung topisch auf die Haut aufgetragen wird.


**Claims**

1. An active substance combination for the artificial tanning of skin, containing a combination of erythrulose in D- or L-form or also as a racemate, with an additional reducing sugar having self-tanning properties.

2. An active substance combination according to claim 1, **characterized in that** the ratio between erythrulose and the additional reducing sugar having self-tanning properties lies between 10 : 1 and 1 : 10, preferably at about 1 : 1 to 1 : 3, ca. 1 : 2 being particularly preferred.

3. An active substance combination according to claim 1 or 2, **characterized in that** DHA is used as the additional reducing sugar having self-tanning properties.

4. A cosmetic formulation containing an active substance combination according to one of claims 1 or 2, **characterized in that** 1-5% of erythrulose and 1-15% of an additional reducing sugar having self-tanning properties, preferably about 1.5% of erythrulose and 3.5% of an additional reducing sugar having self-tanning properties, are used.

5. A cosmetic formulation containing an active substance combination according to one of claims 1 or 2, **characterized in that** DHA is used as the additional reducing sugar having self-tanning properties.

6. A cosmetic formulation according to one of claims 4 or 5 in the form of an O/W emulsion.

7. A cosmetic formulation according to claim 6, **characterized in that** the oily phase amounts to 5-45% by weight, in particular about 10-30% by weight of the total formulation.

8. A cosmetic formulation according to one of claims 4 - 7, **characterized in that** it contains at least one penetration enhancer.

9. A cosmetic formulation according to one of claims 4 - 8, **characterized in that** it contains at least one compound acting as a sunscreen, preferably octyl methoxycinnamate and/or butyl methoxydibenzoylmethane.

10. A non-therapeutic method for the topical use of an active substance combination according to one of claims 1-3 or of a cosmetic formulation according to one of claims 4-9, comprising topically applying an effective quantity of an active substance combination or of a cosmetic formulation on the skin.

11. Use of an active substance combination according to one of claims 1-3 for the preparation of a cosmetic formulation according to one of claims 4-9.

12. Use of a cosmetic formulation according to one of claims 4-9 for the artificial tanning of skin, the formulation being topically applied on the skin.

**Revendications**

1. Combinaison de principes actifs pour la coloration artificielle de la peau comprenant une association d'érythrulose de conformation D, L ou racémique, avec un autre sucre réducteur ayant des propriétés autobronzantes.

2. Combinaison de principes actifs selon la revendication 1, **caractérisée en ce que** le rapport entre l'érythrulose et l'autre sucre réducteur ayant des propriétés autobronzantes soit compris entre 10 : 1 et 1 : 10, de préférence entre 1 : 1 et 1 : 3, et particulièrement autour de 1 : 2.

3. Combinaison de principes actifs selon la revendication 1 ou 2, **caractérisée en ce que** la DHA soit utilisée comme autre sucre réducteur ayant des propriétés autobronzantes.

4. Formulation cosmétique comprenant une combinaison de principes actifs selon l'une des revendications 1 ou 2, **caractérisée en ce que** l'on y incorpore 1-5% d'érythrulose et 1-15% d'un autre sucre réducteur ayant des propriétés autobronzantes, de préférence environ 1,5% d'érythrulose et 3,5% d'un autre sucre réducteur ayant des propriétés autobronzantes.

5. Formulation cosmétique comprenant une combinaison de principes actifs selon l'une des revendications 1 ou 2, **caractérisée en ce que** la DHA soit utilisée comme autre sucre réducteur ayant des propriétés autobronzantes.

6. Formulation cosmétique selon l'une des revendications 4 ou 5 sous la forme d'une émulsion H/E.

7. Formulation cosmétique selon la revendication 6, **caractérisée en ce que** la phase huileuse s'élève à 5-45% en poids, en particulier à 10-30% en poids de la formulation totale.

8. Formulation cosmétique selon l'une des revendications 4-7, **caractérisée en ce qu'**elle contienne au moins un promoteur d'absorption.

9. Formulation cosmétique selon l'une des revendications 4-8, **caractérisée en ce qu'**elle contienne au moins un composé agissant comme un filtre solaire, de préférence de l'octylméthoxycinnamate et/ou du butylméthoxydibenzoylméthane.

10. Méthode non thérapeutique pour l'application topique d'une combinaison de principes actifs selon l'une des revendications 1-3 ou d'une formulation cosmétique selon l'une des revendications 4-9, **caractérisée en ce qu'**une quantité efficace d'une combinaison de principes actifs ou d'une formulation cosmétique soit appliquée topiquement sur la peau.

11. Utilisation d'une combinaison de principes actifs selon l'une des revendications 1-3 pour la préparation d'une formulation cosmétique selon l'une des revendications 4-9.

12. Utilisation d'une formulation cosmétique selon l'une des revendications 4-9 pour la coloration artificielle de la peau, la formulation étant appliquée sur la peau de manière topique.

**Fig. 1**

**Fig. 2**